# EUROPEAN PATENT SPECIFICATION

(11) **EP 2 444 385 B1**
(45) Date of publication and mention of the grant of the patent: **10.12.2014**
(21) Application number: 10789480.0
(22) Date of filing: 15.06.2010
(51) Int. Cl.: C07C 41/05, C07C 41/58, C07C 43/12, C07B 61/00

(54) **METHOD FOR PRODUCING FLUORINE-CONTAINING ETHER WITH HIGH PURITY**
VERFAHREN ZUR HERSTELLUNG EINES FLUORHALTIGEM ETHERS VON HOHER REINHEIT
PROCÉDÉ POUR LA PRODUCTION D'ÉTHER CONTENANT DU FLUOR DE PURETÉ ÉLEVÉE

(30) Priority: 15.06.2009 JP 2009142402
(43) Date of publication of application: 25.04.2012
(73) Proprietor: Daikin Industries, Ltd., Osaka-shi, Osaka 530-8323 (JP)
(72) Inventor: KAGAWA, Michiru, Settsu-shi Osaka 566-8585 (JP); NAKAZONO, Aoi, Settsu-shi Osaka 566-8585 (JP); SENBA, Yasuhide, Settsu-shi Osaka 566-8585 (JP); MOTOYAMA, Hitoshi, Settsu-shi Osaka 566-8585 (JP); YOSHIZAWA, Toru, Settsu-shi Osaka 566-8585 (JP); KOH, Meiten, Settsu-shi Osaka 566-8585 (JP)
(74) Representative: Hoffmann Eitle
(86) International application number: PCT/JP2010/060085
(87) International publication number: WO 2010/147105

(56) References cited:
- EP-A1- 1 346 757
- WO-A2-2007/019161
- WO-A2-2009/085247
- JP-A- H07 505 899
- JP-A- 2002 047 218
- JP-A- 2002 201 152
- JP-A- 2006 306 726
- JP-A- 2008 230 981

## Description

### TECHNICAL FIELD

The present invention relates to a method for producing a highly pure fluoroether by simple purification.

### BACKGROUND ART

It is known that fluoroethers are synthesized by reacting fluorine-containing alkyl alcohols with fluorinated olefins in the presence of basic compounds, such as alkali metal compounds (Patent Documents 1 to 6). However, small amounts of fluorine-containing alkyl alcohols as raw materials and by-products remain in the reaction products (a fluoroether reaction crude solution).

Impurities (particularly fluorine-containing alkyl alcohols) are separated (removed by purification) from a fluoroether reaction solution by rinsing the solution with water and distillation of the solution (rectification or simple distillation), or by performing these operations in combination. However, the purity of the fluoroether after the purification is estimated not to exceed the range of about 91.0 to 99.0% based on the Patent Documents.

Further, it is suggested that unsaturated impurities generated as by-products are removed as chlorine adducts which are obtained by adding chlorine to the unsaturated compound (Patent Documents 7 to 9). However, such a method does not allow the separation of fluorine-containing alkyl alcohols, and further the method needs to be performed using light and/or performed under high temperature. Therefore, the problem arises that equipment such as a high-pressure mercury-vapor lamp is needed and the number of processing steps increases.

Patent Document 1: JP-H09-263559A
Patent Document 2: JP-2002-201152A
Patent Document 3: JP-2004-345967A
Patent Document 4: JP-2005-068142A
Patent Document 5: JP-2005-132826A
Patent Document 6: JP-2005-306800A
Patent Document 7: WO 2006/123563
Patent Document 8: JP-2008-230979A
Patent Document 9: JP-2005-230981A

WO 2007/019161 discloses the purification of fluoromethyl 1,1,1,3,3,3-hexafluoroisopropyl ether (sevoflurane) by combining a crude sevoflurane product with sufficient water to produce a multiphase mixture, fractionally distilling the multiphase mixture, and removing substantially pure sevoflurane from the distilling multiphase mixture.

### SUMMARY OF THE INVENTION

### PROBLEMS TO BE SOLVED BY THE INVENTION

The present inventors have noted that the target fluoroether needs to have very high purity when used as electrolytes used for cells, polymer solvents and detergents used for semiconductors. The inventors have examined a method for certainly and efficiently separating an impurity fluorine-containing alkyl alcohol as a raw material from a crude solution of fluoroether. They tried various separation methods such as a rectifying method (fractional distillation), a crystallization method, a rinsing method (rinsing with water and removing a water phase).

However, the fluoroether and the fluorine-containing alkyl alcohol as a raw material are likely to form an azeotropic system, and they are therefore hardly separated by a rectification method. Further, a crystallization method in which a substance is crystallized in water using a difference in a freezing point is hardly used for the separation of the fluorine-containing alkyl alcohol because the fluorine-containing alkyl alcohol is not frozen at the freezing point thereof.

In the rinsing method, the fluorine-containing alkyl alcohol is eluted in water by rinsing the crude solution of fluoroether with water. As a result, the concentration of the fluorine-containing alkyl alcohol can be reduced to the best (about 0.001% or less, gas chromatography concentration, hereinafter referred to GC%) .

However, in order to reduce the concentration of the fluorine-containing alkyl alcohol to 0.001 GC% or less, the rinsing needs to be repeated many times using a large amount of water. For example, in order to reduce the concentration of the fluorine-containing alkyl alcohol from about 9 GC% to 0.001 GC% or less, the rinsing needs to be repeated 6 times, and about 4 times as much water as the crude solution of fluoroether is needed. Therefore, the rinsing method is disadvantageous for scale expansion.

The present invention has an object to provide a method for efficiently producing a highly pure fluoroether suitable for scale expansion, and the concentration of a fluorine-containing alkyl alcohol thereof is remarkably reduced.

### MEANS FOR SOLVING THE PROBLEMS

That is, the present invention relates to a method for producing a highly pure fluoroether, the method comprising: performing countercurrent separation on a crude solution of fluoroether including a fluorine-containing alkyl alcohol as an impurity using water, wherein the crude solution of fluoroether as a starting material is a reaction solution prepared by reacting a fluorine-containing alkyl alcohol with a fluorinated olefin in the presence of a basic compound.

According to the present invention, the highly pure fluoroether including a fluorine-containing alkyl alcohol in a concentration of 0.001 GC% or less can be prepared from the crude solution of fluoroether including a fluorine-containing alkyl alcohol in a concentration of 0.2 to 20 GC%.

In the present invention, the fluoroether is, for example, a fluorine-containing alkyl ether represented by formula (3):

RfCH₂OCF₂CHXY (3)

wherein Rf is a fluorine-containing alkyl group, X and Y are the same as or different from each other, and are each a hydrogen atom, a chlorine atom, a fluorine atom, or a trifluoro methyl group.

The fluorine-containing alkyl alcohol according to the present invention is, for example, a compound represented by formula (1):

RfCH₂OH (1)

wherein Rf is a fluorine-containing alkyl group.

The fluorinated olefin used for the synthesis of the fluoroether is, for example, a compound represented by formula (2):

CF₂=CXY (2)

wherein X and Y are the same as or different from each other, and are each a hydrogen atom, a chlorine atom, a fluorine atom, or a trifluoro methyl group.

The present invention relates to a method for producing a highly pure fluoroether comprising adjusting a moisture content of the fluoroether prepared through the countercurrent separation to 50 ppm or less and/or distilling the fluoroether.

### EFFECT OF THE INVENTION

The production method of the present invention can provide a method for efficiently producing a highly pure fluoroether suitable for scale expansion.

A fluoroether phase and a water phase containing an unreacted raw material, which are prepared by the reaction, are easily separated, and the unreacted raw material in the water phase is reusable. Therefore, such a method provides advantages in view of reduction in costs and mass production.

Further, a fluoroether can be purified to be of high purity by (simple) distillation.

The highly pure fluoroether in which a moisture content is reduced is particularly useful as e.g. a component of an electrolyte used for a cell or a capacitor.

### MODES FOR CARRYING OUT THE INVENTION

The method for producing a highly pure fluoroether of the invention comprises performing countercurrent separation on a crude solution of fluoroether including a fluorine-containing alkyl alcohol as an impurity using water.

The crude solution of fluoroether (as a starting material) is a reaction product solution prepared by using a fluorine-containing alkyl alcohol with a fluorinated olefin in the presence of a basic compound. Patent Documents 1 to 9 disclose the reaction.

Specifically, preferred is a fluorine-containing alkyl alcohol represented by formula (1):

RfCH₂OH (1)

wherein Rf is a fluorine-containing alkyl group, because of its good nucleophilic addition reactivity of the fluorine-containing alkyl alcohol to a fluorinated olefin.

The fluorine-containing alkyl group represented by Rf is a group in which at least one of hydrogen atoms of an alkyl group is displaced by a fluorine atom. The fluorine-containing alkyl group may be a chain or branched C1-C8 fluorine-containing alkyl group. The fluorine-containing alkyl group preferably has 1 to 6 carbon atoms, and more preferably has 1 to 4 carbon atoms.

Specific examples of the fluorine-containing alkyl group include CF₃-, CF₃CF₂-, CF₃(CF₂)₂-, CF₃(CF₂)₃-, CF₃(CF₂)₄-, CF₃(CF₂)₅-, CHF₂CF₂-, CHF₂(CF₂)₃-, CHF₂(CF₂)₅-, (CF₃)₂CF-, and (CF₃)₂CH-. CF₃-, CF₃CF₂-, or CHF₂CF₂- is preferred.

In view of the good reactivity of such a compound, the fluorinated olefin, which is one of the raw materials, is a compound represented by formula (2):

CF₂=CXY (2)

wherein X and Y are the same as or different from each other, and are each a hydrogen atom, a chlorine atom, a fluorine atom, or a trifluoro methyl group.

Specific examples of the fluorinated olefin include CF₂=CF₂, CF₂=CHF, CF₂=CH₂, CF₂=CFCl, and CF₂=CFCF₃. CF₂=CF₂, CF₂=CFCl, and CF₂=CFCF₃ are preferred in view of the good reactivity.

The fluorinated olefin and the fluorine-containing alkyl alcohol are reacted in equimolar amounts. However, the conversion of the fluorine-containing alkyl alcohol needs to be reduced up to 70%. Therefore, an actual amount of the fluorinated olefin to be introduced into the reaction system is preferably 1.0 mol or less, more preferably 0.7 mol or less, and particularly preferably 0.65 mol or less; and preferably 0.3 mol or more, and more preferably 0.5 mol or more, relative to 1 mol of the fluorine-containing alkyl alcohol, considering that the amount of the fluorinated olefin discharged as an unreacted material after the reaction.

The basic compound acts as a catalyst in the production method of the present invention, and is preferably an inorganic basic compound because it easily forms an alkoxide with the fluorine-containing alkyl alcohol. Particularly, alkali metal hydroxides such as NaOH, KOH, CsOH, and LiOH, or alkali earth metal hydroxides such as Ca(OH)₂ and Ba(OH)₂ are more preferred in view of the good dissociation. The amount of the basic compound is preferably 0.01 mol or more, more preferably 0.2 mol or more, and particularly preferably 0.3 mol or more; and preferably 1.0 mol or less, and more preferably 0.8 mol or less, relative to 1 mol of the fluorine-containing alkyl alcohol, in view of a rate of reaction and/or good selectivity of the fluoroether. In the reaction of the basic compound with the fluorine-containing alkyl alcohol, heat of reaction may be generated to cause abnormal elevation of the temperature, and/or an alkoxide formed in the reaction may be explosive. Therefore, in view of safety, the basic compound is used as a 5 to 40% by mass aqueous liquid, and preferably used as a 15 to 25% by mass aqueous liquid.

The reaction of the fluorine-containing alkyl alcohol with the fluorinated olefin is preferably generally carried out in a solvent. Examples of the solvent include water and polar organic solvents such as diethyl ether, glymes, dioxane, tetrahydrofuran, and acetonitrile. In the present invention, water is preferably used because water tends to prevent generation of a by-product having an unsaturated bond and the target fluoroether is easily separated from water. As the water, ion exchange water or distilled water including less impurity is preferably used.

The reaction is carried out under ordinary pressure or under pressure because the fluorinated olefin as a raw material is a gas at ordinary temperatures. In view of a decrease in the rate of progress of a side reaction and an increase in the purity of the fluoroether, the reaction pressure is preferably 0.05 MPa or more, more preferably 0.2 MPa or more, and particularly preferably an absolute pressure of 0.4 MPa or more; and is preferably 1.0 MPa or less, more preferably 0.85 MPa or less, and particularly preferably 0.8 MPa or less. The reaction is carried out at a temperature in the range from 25 to 90°C, and preferably 50 to 85°C.

The fluoroether produced in the reaction is a fluorine-containing alkyl ether represented by formula (3) when the compounds represented by formulae (1) and (2) are used as raw materials.

RfCH₂OCF₂CHXY (3)

In the formula, Rf, X, and Y are the same as defined above.

The resulting fluorine-containing alkyl ether reaction product solution includes an unreacted fluorine-containing alkyl alcohol as described above. The reaction product solution may further include an unsaturated by-product produced by a dehydrohalogenation reaction caused by a basic compound in the reaction system.

The yield and purity (concentration) of the fluoroether of the resulting reaction product solution, and the amount of impurities change depending on reaction conditions. The fluoroether concentration of the crude solution of fluoroether of the present invention is preferably 80 GC% or more, more preferably 85 GC% or more, and particularly preferably 90 GC% or more in order to reduce the number of steps of the countercurrent separation and to further reduce water consumption. The upper limit of the fluoroether concentration of the crude solution is desirably as high as possible. However, the concentration is 99.8 GC% or less at the best. The fluoroether concentration of the reaction product solution cannot be increased to 99.8 GC% only by the synthesis reaction. Therefore the reaction product solution generally needs to undergo some purification. In the present invention, purification is performed with good efficiently, and therefore, the fluoroether concentration may be 85 GC% or less, and 90 GC% or less.

In the present invention, the countercurrent extraction on the crude solution of fluoroether (as a starting material) is performed using water as an extraction solvent (separating agent) for the fluorine-containing alkyl alcohol.

The countercurrent extraction method is a liquid-liquid extraction method, and the extraction is performed using a vertical extraction device. The method includes: pouring a crude solution of fluoroether having a large gravity (for example, the gravity is about 1.5) into the extraction device from the upper portion thereof; pouring water (gravity is 1.0) from the lower portion; and making water droplets float toward an upper portion of the device with stirring of the solution if needed. While the water droplets are floating, the crude solution of fluoroether is efficiently brought into contact with water, and the fluorine-containing alkyl alcohol is extracted with the water droplets. The water used for the extraction is withdrawn from the upper portion of the device.

A mixer-settler type extraction device having a multiple-stage stirrer is a typical countercurrent extraction device.

For example, Patent Documents JP-2001-39962A, JP-7-188085A, and JP-5-170755A disclose a separation method by countercurrent extraction. However, a method for separating a fluorine-containing compound by countercurrent extraction has never known.

The fluorine-containing alkyl alcohol concentration of the crude solution of fluoroether is desirably low. According to the method of the present invention, the crude solution can be purified to a fluorine-containing alkyl alcohol concentration of 0.001 GC% or less even from 0.2 to 20 GC%. The fluorine-containing alkyl alcohol concentration is preferably 0.2 to 20 GC%, more preferably 1 to 15 GC%, and particularly preferably 1 to 10 GC%, in view of the number of steps of countercurrent separation and water consumption.

The number of steps, the stirring speed, and the diameter of the column may be appropriately selected for the countercurrent extraction device in accordance with the fluoroether concentration of the crude solution of fluoroether and/or a target purity. For example, in the case where a crude solution of fluoroether having a fluorine-containing alkyl alcohol concentration of 9 GC% (the fluoroether concentration is 90 GC%) is purified by a 24-step countercurrent extraction method, the fluorine-containing alkyl alcohol concentration can be reduced to 0.001 GC% or less. (in such a case, the fluoroether concentration is about 99.6 GC%) In order to reduce a similar level of the fluorine-containing alkyl alcohol concentration to 0.001 GC% or less by a rinsing method (operation of rinsing with water and removing a water phase), the operations of rinsing with water and removing a water phase need to be repeated six times. Therefore, the amount of water needed for the rinsing with water is four times as much as that used in the countercurrent extraction method.

The number of steps is preferably five or more and more preferably 10 or more in view of the separation capacity. The separation capacity is enhanced as the number of steps increases, but the device grows in size. Therefore, the number of steps is preferably 50 or less. The stirring speed is desirably high in order to increase a liquid-liquid contact area, and is desirably 20 rpm or more. However, if the stirring speed is too high, bubbles may generate in the liquid, which leads to decrease in contact efficiency. Therefore, the stirring speed is desirably 4000 rpm or less. Heavy liquid feed rate/light liquid feed rate changes depending on the diameter of the column. In order to obtain a certain processing capacity, 10 kg or more per one hour is desirable. The process is performed using water, and therefore the processing temperature is desirably 2°C or more, and desirably 90°C or less.

The countercurrent extraction may be performed two or more times. Before the countercurrent extraction is performed, the fluoroether reaction product solution may be rinsed with water in a usual manner to prepare the crude solution of fluoroether with water-soluble compounds such as fluorine-containing alkyl alcohols and basic compounds reduced to some extent.

In the countercurrent extraction, the basic materials in addition to fluorine-containing alkyl alcohols are soluble in a water phase, and therefore can be removed by separating the water phase.

An alcohol content can be calculated by a specific gravity method from the water phase taken out from the countercurrent extraction device. Therefore the phase may be reused for the synthesis of the fluoroether.

Thus, according to the countercurrent extraction in the present invention, the amount of water can be remarkably reduced and the processing time can be reduced. Further, the fluorine-containing alkyl alcohols and/or the basic compounds can be easily removed from the water phase in a short time because the amount of the water phase is small.

Further, the fluoroether can be produced with less environmental impact because the most of the production steps thereof including the countercurrent extraction and the (simple) distillation described below are performed in a closed system. After the fluoroether is synthesized, subsequent steps to purification may be continuously performed.

Unsaturated compounds are one of by-products insoluble in the water phase. The concentration of such unsaturated compounds of the reaction product solution is less than 0.3 GC% at the best. There is little difference between the boiling points of the unsaturated compounds and the ether. Therefore, the concentration of the unsaturated compounds is extremely difficult to be reduced to 0.01 GC% or less by distillation. However, if included in the ether, the unsaturated compounds have particularly no effect on the physical properties of the ether.

The highly pure fluoroether obtained in the present invention is useful, for example, as a component (solvent) of an electrolyte used for a lithium secondary battery and a capacitor, various solvents, and a detergent for a semiconductor. Particularly, when the highly pure fluoroether is used as solvents of an electrolyte, the moisture content is desirably reduced to 50 ppm or less, preferably 30 ppm or less, and more preferably 10 ppm or less.

Examples of the method for reducing the moisture content include: adsorbing moisture using a dehydrating agent such as molecular sieves; and azeotropic distillating using azeotropy solvents such as hexane. Such methods may be used in the present invention. Fluoroether and water form a heterogeneous azeotrope, and therefore the fluoroether countercurrent-extracted is preferably directly (continuously) distilled in view of costs.

### EXAMPLE

The present invention is explained based on Examples and Comparative Examples in the following, but the invention is not limited only thereto.

The measuring method used in the present invention is described below.

### (1) Composition analysis

NMR method: AC-300 which was a product of Bruker Corporation was used.

### ¹⁹F-NMR:

Measuring condition: 282 MHz (trichlorofluoromethane = 0 ppm)

### ¹H-NMR:

Measuring condition: 300 MHz (tetramethylsilane = 0 ppm)

### (2) Concentration (GC%) analysis

Gel-chromatography (GC) method: GC-17A produced by SHIMADZU CORPORATION was used. Column: DB624 (Length 60, I.D 0.32, Film 1.8 micrometers) was used. Measurement limit: 0.001%

### (3) Moisture content (% by mass or ppm)

Karl Fischer method: MKC-501 which is a product of Kyoto Electronics Manufacturing Co., Ltd. was used. Catholyte: AQUAMICRON CXU which is a product of Mitsubishi Chemical Corporation
Anolyte: AQUAMICRON AKX which is a product of Mitsubishi Chemical Corporation

### Synthesis Example 1 (production of a crude solution of fluoroether)

A system of a 6-L autoclave made of stainless steel was evacuated. The autoclave was charged with 401 g of potassium hydroxide (7.15 mol, 0.55 molar amount relative to 1 mol of a fluorine-containing alkyl alcohol), water (1604 mL), 2,2,3,3-tetrafluoro-1-propanol (boiling point of 109°C, specific gravity of 1.4) represented by HCF₂CF₂CH₂OH (1716 g, 13 mol) as a fluorine-containing alkyl alcohol. Then, the system was evacuated and replaced with nitrogen 20 times at room temperature. After evacuated, the system was filled with tetrafluoroethylene to 0.1 MPa, and the reaction system was heated to 85°C. After the temperature in the system reached 85°C, tetrafluoroethylene was added thereto little by little so that the reaction pressure was kept at 0.5 to 0.8 MPa. The temperature in the system was adjusted so as to be kept at 75 to 95°C.

Tetrafluoroethylene was added until the amount reached 0.5 molar relative to 1 mol of the fluorine-containing alkyl alcohol. The reaction was continuously carried out under stirring. When reduction in the pressure in the autoclave was stopped, the temperature in the autoclave was reduced to room temperature and unreacted tetrafluoroethylene was discharged, and the reaction was stopped. These steps took 5 hours to perform.

The fluoroether of a lower phase of the production solution was HCF₂CF₂CH₂OCF₂CF₂H (boiling point of 92°C, specific gravity of 1.52). Table 1 shows the composition of the fluoroether production solution of a lower phase resulting from GC analysis.

### Synthesis Example 2

The fluoroether was synthesized in the same way as synthesis example 1, except that the amount of tetrafluoroethylene was 780 g (7.8 mol). Table 1 shows the composition of the obtained fluoroether production solution.

### Synthesis Example 3

The fluoroether was synthesized in the same way as synthesis example 1, except that the amount of tetrafluoroethylene was 910 g (9.1 mol). Table 1 shows the composition of the obtained fluoroether production solution.

### Synthesis Example 4

The fluoroether was synthesized in the same way as synthesis example 1, except that the amount of tetrafluoroethylene was 1040 g (10.4 mol). Table 1 shows the composition of the obtained fluoroether production solution.

### Synthesis Example 5

The fluoroether was synthesized in the same way as synthesis example 1, except that the amount of tetrafluoroethylene was 1170 g (11.7 mol). Table 1 shows the composition of the obtained fluoroether production solution.

**[Table 1]**

| Synthesis Example | Concentration of fluoroether (GC%) | Concentration of fluorine-containing alkyl alcohol (GC%) | Others (GC%) |
|---|---|---|---|
| 1 | 98.7 | 1.0 | 0.3 |
| 2 | 90.2 | 8.8 | 0.9 |
| 3 | 88.3 | 10.6 | 1.2 |
| 4 | 83.4 | 15.3 | 1.4 |
| 5 | 78.6 | 20.0 | 1.4 |

### Examples 1 to 5

The amount 1500 g of each of the fluoroether production solutions prepared in synthesis examples 1 to 5 was used as a crude solution of fluoroether. Countercurrent extraction of the crude solution was performed in the following conditions using a mixer-settler type extraction device.
Mixer-settler type extraction device: (column height of 3300 mm, internal diameter of 200 mm)
Number of steps: 24 steps
Stirring speed: 285 rpm
Heavy liquid feed rate: 160 kg/hr
Light liquid: pure water
Light liquid feed rate: 100 kg/hr
Processing temperature: 27°C

The processing time was 0.01 hour. Table 2 shows amounts of water used for the crude solution of fluoroether with the compositions, respectively.

**[Table 2]**

| Example | Crude solution of fluoroether | Concentration of fluorine-containing alkyl alcohol (GC%) | | Amount of water (g/g of fluoroether) |
|---|---|---|---|---|
| | | Before extracting | After extracting | |
| 1 | Synthesis Example 1 | 1.0 | 0.001 or less | 0.567 |
| 2 | Synthesis Example 2 | 8.8 | 0.001 or less | 0.629 |
| 3 | Synthesis Example 3 | 10.6 | 0.001 or less | 0.634 |
| 4 | Synthesis Example 4 | 15.3 | 0.001 or less | 0.645 |
| 5 | Synthesis Example 5 | 20.0 | 0.001 or less | 0.654 |

As shown in Table 2, the fluorine-containing alkyl alcohol concentrations of the fluoroethers extracted were all 0.001 GC% or less, regardless of the difference in the fluorine-containing alkyl alcohol concentration on the crude solutions of fluoroether. The moisture contents of the fluoroethers extracted were all 960 ppm.

Simple distillation of each of the fluoroethers was performed, and a 99.8 GC% of highly pure fluoroether was prepared as a fraction obtained at 92°C. The moisture content was 10 ppm or less. The yield of the highly pure fluoroether from the crude solution of fluoroether was 90%.

### Synthesis Example 6

Fluoroether represented by HCF₂CF₂CH₂OCF₂CFHCF₃ (boiling point of 108°C, specific gravity of 1.61) was synthesized in the same way as synthesis example 1, except that hexafluoropropylene was used instead of tetrafluoroethylene.

GC analysis of the fluoroether phase, which was a lower phase of the resulting production solution, shows that the concentration of the fluoroether was 97.1GC%, the concentration of the unreacted fluorine-containing alkyl alcohol was 2 GC%, and the concentration of other components (unsaturated compound of a by-product and an unidentified product) was 0.9 GC%.

### Example 6

The amount 1500 g of the fluoroether production solution prepared in synthesis example 6 was used as the crude solution of fluoroether, and countercurrent extraction of the crude solution was performed in the following conditions using a mixer-settler type extraction device.
Mixer-settler type extraction device: (column height of 3300 mm, internal diameter of 200 mm)
Number of steps: 24 steps
Stirring speed: 285 rpm
Heavy liquid feed rate: 160 kg/hr
Light liquid: pure water
Light liquid feed rate: 100 kg/hr
Processing temperature: 27°C

The processing time was 0.01 hour. The water amount was 0.62 g per 1 g of the fluoroether. The fluorine-containing alkyl alcohol concentration of the resulting fluoroether was 0.001 GC% or less, the fluoroether concentration was 99.8 GC%, and the moisture content was 960 ppm.

Simple distillation of the fluoroether was performed, and a 99.8 GC% highly pure fluoroether was prepared as a fraction obtained at 108°C. The moisture content was 10 ppm or less. The yield of the highly pure fluoroether from the crude solution of fluoroether was 95%.

### Comparative Example 1 (separation by distillation)

The crude solution of fluoroether prepared in the synthesis example was in vapor-liquid equilibrium and was inseparable in a fluorine-containing alkyl alcohol dilute zone (concentration of 8.839 GC%). The fluoroether and the fluorine-containing alkyl alcohol formed an azeotrope (fluoroether/fluorine-containing alkyl alcohol = 97/3 mol%). For confirmation, separation by batch distillation was examined. The separation by the batch distillation was hardly achieved.

Further, the separation by distillation using the minimum azeotropy of water/alcohol (azeotropic composition: 70/30 GC%, 94°C, 1 atmosphere) was examined. However, the separation by such a distillation was hardly achieved.

### Comparative Example 2 (separation by crystallization)

A 100-mL three-necked flask was charged with 39.0 g of the fluoroether represented by HCF₂CF₂CH₂OCF₂CF₂H, 2.53 g of the fluorine-containing alkyl alcohol represented by HCF₂CF₂CH₂OH, and 1.33 g of water. Thus, 42.85 g of a test crude solution of fluoroether (composition ratio: fluoroether 91 GC%/fluorine-containing alkyl alcohol 5.9 GC%/water 3.1% by mass) was prepared.

The crude solution of fluoro ether was slowly stirred with a magnetic stirrer, and the solution was cooled to - 18°C for two hours. The solution was filtered to remove a solid deposition. The resulting filtrate was analyzed for a fluorine-containing alkyl alcohol concentration GC% by gas chromatography, and analyzed for a moisture content by the Karl Fischer technique. The results were that the composition of the filtrate was fluoroether 94.0 GC%/fluorine-containing alkyl alcohol 5.8 GC%/water 0.16% by mass. The proportion of the fluorine-containing alkyl alcohol changed little.

The fluoroether, the fluorine-containing alkyl alcohol, and water, which are three components in the crude solution of fluoroether, have freezing points of lower than -40°C, -15°C, and 0°C, respectively. However, although the temperature of the crude solution of fluoroether was decreased to -18°C, only the separated moisture froze, and the dissolved fluorine-containing alkyl alcohol did not freeze. Therefore, the separation of the fluorine-containing alkyl alcohol was not able to be achieved.

Comparative Example 3 (rinsing with water and removing a water phase)

A rinse treatment of 1500 g (987 mL) of the crude solution of fluoroether prepared in the synthesis example was performed repeatedly so that the concentration of the fluorine-containing alkyl alcohol of the solution was reduced to 0.001 GC% or less (it took about 6 hours). A single rinse treatment includes rinsing with water in the same volume 987 mL as that of the crude solution of fluoroether, and removing the water. Table 3 shows the results.

**[Table 3]**

| The number of rinsing with water | Concentration of fluoroether (GC%) | Concentration of fluorine-containing alkyl alcohol (GC%) | Amount of water (g) |
|---|---|---|---|
| Before rinsing with water | 90.2 | 8.8 | - |
| 1 | 94.4 | 5.2 | 987 |
| 2 | 97.9 | 1.7 | 987 |
| 3 | 99.1 | 0.6 | 987 |
| 4 | 99.5 | 0.2 | 987 |
| 5 | 99.6 | 0.1 | 987 |
| 6 | 99.6 | 0.001 or less | 987 |

As shown in Table 3, 5922 g of water was used to reduce the concentration of the fluorine-containing alkyl alcohol in 1500g of the crude solution of fluoroether to 0.001 GC% or less (4 g per 1 g of the crude solution of fluoroether).

### Example 7

A fluoroether was synthesized by the same method as that of synthesis example 1, except that CF₃CF₂CH₂OH was used instead of the fluorine-containing alkyl alcohol as a raw material and NaOH was used instead of KOH.

The fluoroether, which was the lower phase of the resulting production solution, was CF₃CF₂CH₂OCF₂CF₂H (boiling point of 68°C). The GC analysis of the fluoroether production solution, which was the lower phase of the resulting production solution, showed the composition in which the concentration of the fluoroether was 98.2 GC%, the concentration of the fluorine-containing alkyl alcohol was 1.5 GC%, and the concentration of other components was 0.3 GC%.

Countercurrent extraction of the fluoroether production solution was performed in the same conditions as those in Example 1. As a result, the concentration of the fluorine-containing alkyl alcohol was able to be reduced to 0.0001 GC%.

### Example 8

A fluoroether was synthesized by the same method as that of synthesis example 1, except that CF₃CH₂OH was used instead of the fluorine-containing alkyl alcohol as a raw material and NaOH was used instead of KOH.

The fluoroether, which was the lower phase of the resulting production solution, was CF₃CH₂OCF₂CF₂H (boiling point of 56°C). The GC analysis of the fluoroether production solution, which was the lower phase of the resulting production solution, showed the composition in which the concentration of the fluoroether was 98.2 GC%, the concentration of the fluorine-containing alkyl alcohol was 1.3 GC%, and the concentration of other components was 0.5 GC%.

Countercurrent extraction of the fluoroether production solution was performed in the same conditions as those in Example 1. As a result, the concentration of the fluorine-containing alkyl alcohol was able to be reduced to 0.0001 GC%.

### Example 9

A fluoroether CF₃CF₂CH₂OCF₂CFHCF₃ (boiling point of 87°C) was synthesized by carrying out the same reaction as that of Example 7, except that hexafluoropropylene was used instead of tetrafluoroethylene.

The GC analysis of the fluoroether phase, which was the lower phase of the resulting production solution, showed that the concentration of the fluoroether was 97.6 GC%, the concentration of unreacted fluorine-containing alkyl alcohol was 2.2 GC%, and the concentration of other components (unsaturated compound of a by-product and an unidentified product) was 0.2 GC%.

Countercurrent extraction of the fluoroether production solution was performed in the same conditions as those in Example 1. As a result, the concentration of the fluorine-containing alkyl alcohol was able to be reduced to 0.0001 GC%.

### Example 10

A fluoroether was synthesized by the same method as that of synthesis example 1, except that CF₃CF₂CH₂OH was used instead of the fluorine-containing alkyl alcohol as a raw material and chlorotrifluoroethylene was used instead of tetrafluoroethylene.

The fluoroether, which was the lower phase of the resulting production solution, was HCF₂CF₂CH₂OCF₂CFClH (boiling point of 108°C). The GC analysis of the fluoroether production solution, which was the lower phase of the resulting production solution, showed the composition in which the concentration of the fluoroether was 98.3 GC%, the concentration of the fluorine-containing alkyl alcohol was 1.2 GC%, and the concentration of other components was 0.5 GC%.

Countercurrent extraction of the fluoroether production solution was performed in the same conditions as those in Example 1. As a result, the concentration of the fluorine-containing alkyl alcohol was able to be reduced to 0.0001 GC%.

## Claims

1. A method for producing a highly pure fluoroether, comprising performing countercurrent separation on a crude solution of fluoroether including a fluorine-containing alkyl alcohol as an impurity using water,
wherein the crude solution of fluoroether is a reaction solution prepared by reacting a fluorine-containing alkyl alcohol with a fluorinated olefin in the presence of a basic compound.

2. The method of Claim 1, wherein
- the crude solution of fluoroether includes a fluorine-containing alkyl alcohol in a concentration of 1-20 GC%, and
- the highly pure fluoroether includes a fluorine-containing alkyl alcohol in a concentration of ≤ 0.001 GC%.

3. The method of Claim 1 or 2, wherein
- the fluorine-containing alkyl alcohol is a compound of the formula RfCH₂OH (1), wherein Rf is a fluorine-containing alkyl group;
- the fluorinated olefin is a compound of the formula CF₂=CXY (2), wherein X and Y each independently are H, Cl, F or CF₃; and
- the fluoroether is a compound of the formula RfCH₂-O-CF₂CHXY (3), wherein Rf is fluoroalkyl, and X and Y each independently are H, Cl, F or CF₃.

4. The method of any of claims 1-3, which further comprises the step of distilling the fluoroether produced.

5. The method of any of claims 1-4, which further comprises the step of adjusting the moisture content of the fluoroether produced to ≤ 50 ppm.

## Patentansprüche

1. Verfahren zur Herstellung eines hochreinen Fluorethers, das die Durchführung einer Gegenstromtrennung an einer Fluorether-Rohlösung, die einen fluorhaltigen Alkylalkohol als Verunreinigung enthält, unter Verwendung von Wasser umfasst,
wobei die Fluorether-Rohlösung eine Reaktionslösung ist, die durch Reaktion eines fluorhaltigen Alkylalkohols mit einem fluorierten Olefin in Gegenwart einer basischen Verbindung hergestellt wird.

2. Verfahren gemäß Anspruch 1, wobei
- die Fluorether-Rohlösung einen fluorhaltigen Alkylalkohol in einer Konzentration von 1 bis 20 GC% enthält, und
- der hochreine Fluorether einen fluorhaltigen Alkylalkohol in einer Konzentration von ≤ 0,001 GC% enthält.

3. Verfahren gemäß Anspruch 1 oder 2, wobei
- der fluorhaltige Alkylalkohol eine Verbindung der Formel RfCH₂OH (1) ist, wobei Rf eine fluorhaltige Alkylgruppe ist;
- das fluorierte Olefin eine Verbindung der Formel CF₂=CXY (2) ist, wobei X und Y jeweils unabhängig H, Cl, F oder CF₃ sind; und
- der Fluorether eine Verbindung der Formel RfCH₂-O-CF₂CHXY (3) ist, wobei Rf ein Fluoralkyl ist und X und Y jeweils unabhängig H, Cl, F oder CF₃ sind.

4. Verfahren gemäß einem der Ansprüche 1 bis 3, das ferner einen Schritt umfasst, in dem der hergestellte Fluorether destilliert wird.

5. Verfahren gemäß einem der Ansprüche 1 bis 4, das ferner einen Schritt umfasst, in dem der Feuchtigkeitsgehalt des hergestellten Fluorethers auf ≤ 50 ppm eingestellt wird.

## Revendications

1. Procédé de production d'un éther fluoré hautement pur, comprenant l'exécution d'une séparation à contre-courant sur une solution brute d'éther fluoré incluant un alcool alkylique contenant du fluor comme une impureté en utilisant de l'eau,
dans lequel la solution brute d'éther fluoré est une solution de réaction préparée en faisant réagir un alcool alkylique contenant du fluor avec une oléfine fluorée en présence d'un composé basique.

2. Procédé selon la revendication 1, dans lequel
- la solution brute d'éther fluoré inclut un alcool alkylique contenant du fluor à une concentration de 1 à 20% GC, et
- l'éther fluoré hautement pur inclut un alcool alkylique contenant du fluor à une concentration de ≤ 0,001% GC.

3. Procédé selon la revendication 1 ou 2, dans lequel
- l'alcool alkylique contenant du fluor est un composé de la formule RfCH₂OH (1), dans laquelle Rf est un groupe alkyle contenant du fluor ;
- l'oléfine fluorée est un composé de la formule CF₂=CXY (2), dans laquelle X et Y chacun indépendamment sont H, Cl, F ou CF₃ ; et
- l'éther fluoré est un composé de la formule RfCH₂-O-CF₂CHXY (3), dans laquelle Rf est un alkyle fluoré, et X et Y chacun indépendamment sont H, Cl, F ou CF₃.

4. Procédé selon l'une quelconque des revendications 1 à 3, qui comprend en outre l'étape de distillation de l'éther fluoré produit.

5. Procédé selon l'une quelconque des revendications 1 à 4, qui comprend en outre l'étape d'ajustement de la teneur en humidité de l'éther fluoré produit à ≤ 50 ppm.
